(19) Europäisches Patentamt
European Patent Office
Office européen des brevets

(11) Publication number: 0 224 126 A2

(12) EUROPEAN PATENT APPLICATION

(21) Application number: 86115701.4

(22) Date of filing: 12.11.86

(51) Int.Cl.⁴: C 12 Q 1/68

(30) Priority: 26.11.85 US 801900

(43) Date of publication of application: 03.06.87 Bulletin 87/23

(84) Designated Contracting States: AT BE CH DE ES FR GB GR IT LI LU NL SE

(71) Applicant: The University of Calgary
3330 Hospital Drive
N.W., Calgary Alberta T2N 4N1(CA)

(72) Inventor: Van de Sande, Johan
182 Varsity Estates Drive N.W. Calgary
Alberta T3B 2W9(CA)

(72) Inventor: Kilisch, Bernd W.
D406 1919 University Drive N.W. Calgary
Alberta T2N 4K6(CA)

(72) Inventor: Krawetz, Stephen
B215 33615 49th Street N.E. Calgary
Alberta T3A 2L8(CA)

(72) Inventor: Schoenwaelder, Karl-Heinz
Am feldenbergblick 6
D-6233 Kelkheim (TS)(DE)

(74) Representative: Glawe, Delfs, Moll & Partner
Patentanwälte
Postfach 26 01 62 Liebherrstrasse 20
D-8000 München 26(DE)

(54) **Covalently linked complementary oligodeoxynucleotides as universal nucleic acid sequencing primer linkers.**

(57) Methods and compositions are provided involving universal sequencing primers. The primer is characterized by having a double-stranded region, joined by a uniquely cleavable linkage and capable of being linked to a single strand of DNA. One or two universal linkers may be employed for joining to one or both ends of the ds DNA, whereupon denaturation, two extended single-stranded sequences may be obtained terminating in the universal sequencing primer linker ("splinker"). Each of the strands may then be sequenced by conventional methods employing template recognition.

EP 0 224 126 A2

Croydon Printing Company Ltd

# COVALENTLY LINKED COMPLEMENTARY OLIGODEOXYNUCLEOTIDES AS UNIVERSAL NUCLEIC ACID SEQUENCING PRIMER LINKERS

## BACKGROUND OF THE INVENTION

### Field of the Invention

The sequence analysis of DNA has provided considerable information on gene organization and expression. In conjunction with recombinant DNA technology, sequence analysis has greatly increased our understanding of DNA at the molecular level. Recent advances in sequencing techniques have allowed for the rapid and direct determination of DNA sequences. The current state of this technique is such that DNA sequences can be obtained easier and faster than protein sequences. The latter sequences are now often determined from the DNA sequences.

All present day sequencing approaches depend on the generation of a mixture of single-stranded (ss) DNA molecules differing by one nucleotide of unidirectional length. Gel electrophoretic separation of the DNA molecules of the same sequence but differing in length by as little as one base results in a series of bands of up to approximately 300.

Two basic approaches to DNA sequencing consist of the chemical cleavage method and the enzymatic chain termination approach. The latter method requires that the DNA segment to be sequenced is located adjacent to an available primer site or that a synthetic primer specific for the fragment is available.

The dideoxy nucleotide sequencing of DNA restriction fragments requires either the cloning of the fragment adjacent to a suitable priming site, e.g., M13 (Messing et al., Nucleic Acids Res. (1981) 9:309-321), pBR (Wallace et al., Gene (1981) 16:21-26), or pUC (Vieira and Messing, Gene (1982) 19:259-268) or the availability of a primer specific for that restriction fragment (Smith et al., Cell (1979) 16:753-761; Brenner and Shaw, The EMBO J. (1985) 4:561-568). In contrast, the chemical sequencing strategy can be applied directly to a DNA restriction fragment once an appropriate restriction map has been obtained (Maxam and Gilbert, Proc. Natl. Acad. Sci. USA (1977) 74:560-564).

The dideoxy chain termination procedure (Sanger et al., Proc. Natl. Acad. Sci. USA (1977) 74:5463-5468; Sanger et al., J. Mol. Biol. (1982) 162:729-733) has been very effective in "shotgun" sequence analysis of large DNA fragments. The generation of random DNA fragments by either nuclease digestion or sonication prior to cloning these fragments in an appropriate vector, either single- or double-stranded, has greatly aided this process. Although the sequence data accumulation by the random method is initially very rapid, more directed approaches are usually required to complete the DNA sequence (Bankier and Barrell (1983) In: Techniques in the Life Sciences, B5, Nucleic Acid Biochemistry B508, pp. 1-34 (Elsevier Scientific Publishers Ireland Ltd., County Clare, Ireland).

Description of the Relevant Literature

The finding that T4 DNA ligase can link one strand of unphosphorylated DNA duplex to the 5'-phosphorylated terminus of another segment is reported by Seeburg et al., Nature (1977) 270:486-494 and Lathe et al., DNA (1984) 3:173-182. Loops as short as three or four nucleotides are reported to stabilize short DNA hairpins by Haasnoot et al., J. Biomol. Struct. & Dynamics (1983) 1:115-129. The minimal length of a priming strand for hairpin structures has been shown to be as short as five nucleotides by Kleppe et al., J. Mol. Biol. (1971) 56:241-361. Chain termination sequencing with AMV reverse transcriptase is described by Karathanasis, BRL Focus (1982) 4, no. 3, 6-7.

## SUMMARY OF THE INVENTION

Novel methods and compositions are provided for sequencing DNA. Sequencing primers or "splinkers" are employed which are characterized by having two strands which are at least partially complementary, a cleavable bridge or site, a single strand capable of being covalently joined to a DNA strand and capable of serving as a primer for an enzyme capable of producing a complementary strand from a template single-stranded DNA. In addition, the splinker may be labeled or be capable of being labeled so as to provide a detectable signal. The method also provides for the production of probes.

## DESCRIPTION OF THE SPECIFIC EMBODIMENTS

Methods and compositions are provided for the rapid and efficient sequencing of DNA sequences. The method employs a primer/linker which can be joined to one strand of a double-stranded (ds) DNA fragment. The primer/linker, referred to as a "splinker," is characterized by: generally having fewer than about 100 nucleotides; only one of the strands is capable of forming a covalent linkage with a DNA strand, conveniently as a result of only one strand being phosphorylated; and a sequence of at least 4, usually at least 5 nucleotides will have a complementary sequence (homoduplexed) proximal to the nucleotide to be linked to the strand to be sequenced; the two complementary strands will be joined or crosslinked by a linkage which can be selectively cleaved; the termini at the end where one strand is joined to the fragment may be staggered or flush; the splinker must serve as a useful primer for an enzyme capable of replicating a complementary strand to the single-stranded (ss) fragment joined to the splinker; and the strand to be covalently linked to the fragment to be sequenced may be labeled, where the label may directly or indirectly provide for a detectable signal.

In another sense one may consider the splinker as being a single strand having two complementary regions capable of hybridizing with duplex formation where the individual regions are proximal to the termini of the strand. The complementary regions either include means for forming two strands from the single strand, e.g., the complementary region includes a restriction site, or the complementary regions are joined by a chain which includes a cleavable element, e.g., a restriction site, one or more ribonucleotides, etc. Desirably, the complementary regions are joined by one, preferably at least three nucleotides which remain single-stranded, i.e., do not form base pairs.

Generally, the method will involve providing fragments of ds DNA of interest, which may have blunt or sticky (staggered) ends, linking the splinker to one or opposite ends of the ds fragment, where each splinker is joined only to the 3'-terminus of the ds DNA fragment to be sequenced. This is followed by denaturation to provide a single strand to be sequenced joined at its 3'-terminus to the 5'-terminus of the splinker. The complementary strand is now formed with different mixtures of small dNTP's (deoxyribonucleic acids) and a single ddNTP (dideoxyribonucleic acid). The selective cleavage site of the splinker is then cleaved and the fragments separated on a sequencing gel to determine the sequence.

The splinker will now be considered in detail. The splinker should have a sufficiently long complementary region so as to permit homoduplexing to allow for priming during replication of the complementary strands. Therefore, the splinker will usually be at least about 10 nucleotides, usually at least about 12 nucleotides, more usually at least about 13 nucleotides, preferably at least about 15 nucleotides, more preferably at least about 16 nucleotides. Very large splinkers will usually not provide significant advantages so the splinkers will usually be less than about 100 nucleotides, usually less than about 50 nucleotides, more usually less than about 30 nucleotides, preferably less than about 26 nucleotides, more preferably less than about 20 nucleotides. The complementary region will be at least 4bp, usually at least 5bp and generally fewer than 12bp, more usually 10bp or fewer.

The splinker will conveniently have its 5'-terminus phosphorylated, so that the 5' end of the splinker will be joined to the 3' end of the DNA fragment to be sequenced. While the 5' end of the DNA fragment may have a phosphate group which would serve to link to the 3' end of the splinker, this is not preferred, since the DNA fragments to be sequenced could oligomerize. Since the splinkers may be used in excess, oligomerization of the splinkers is not of concern and will not significantly affect the ultimate sequencing. By removing any terminal phosphate groups with a phosphatase from the DNA fragment to be sequenced, oligomerization of the fragment is prevented and the use of excess splinkers results in the substantially complete joining of the fragments to at least one splinker.

The termini of the splinker may be staggered (sticky) or flush (blunt) depending upon the nature of the terminus of the fragment to which the splinker is to be joined. Thus, where the fragment is formed by mechanical shearing, e.g., sonication or employing a blunt end endonuclease (ENDO R1), the splinker will usually be blunt-ended or by employing an endonuclease that cleaves the two strands at different sites, the splinker will be staggered, where the phosphorylated 5'-terminus will provide any overhang. Where the terminus of the fragment is staggered, usually the splinker will also be staggered, preferably having an overhang which is complementary to at least the last two bases of the overhang of the fragment, preferably being complementary to the overhang of the fragment. The complementary sequence will be the 5'-terminus and will be the site of ligation to the 3'-terminus of one of the strands of the DNA fragment to be sequenced. For example, a -GC overhang of the splinker will be compatible with fragment termini obtained with TaqI, HpaI, ScaNI, AccI, AcyI, AsuII, ClaI and NarI.

The splinker may optionally provide for a label for detection of the primer extended strands when sequencing. The linker need not provide the label, since radioactive nucleotides may be employed for replication of the complementary strand, which would serve to visualize the individual partial strands present in the sequencing gel. However, by providing for a label in the splinker sequence which remains bound to the primer extended strands, namely proximal to the 3'-terminus, this label can be used for detection of the extended partial strands. The label of course may be a radionuclide, such as radioactive phosphorus, nitrogen, oxygen, hydrogen, or the like, or fluorescent nucleotide. Alternatively, ligands may be employed which are covalently bonded to the nucleotide and which may serve as a site for binding of a reciprocal member of a specific binding pair. For example, nucleotides may be covalently linked to biotin, which complexes with avidin in a strong non-covalent link. The avidin may then serve as a site for labels providing detectable signals, such as radionuclides, fluorescers, e.g., fluorescein, rhodamine, phycobiliproteins, umbelliferone, etc.,

enzymes, such as horseradish peroxidase, alkaline phosphatase, uricase, etc. The various labels provide a means for protecting the site of the label, so as to determine the various partial sequences.

In order to allow for the partial sequences to be separated on the gel, the linker must provide for a selectively cleavable functionality. Conveniently, the splinker may provide a rare restriction site, that is, a restriction site not likely to be encountered in the strand being sequenced. Illustrative restriction sites include ClaI, AvaI, HpaI, EcoRI, EcoRV, XbaI, XorII, SmaI, BamHI, BstEII and KpnI. In the event that the splinker is labeled, the restriction site should be upstream from the label, so as to be retained with the partial prime sequence upon restriction with an endonuclease. Besides a restriction site, one or more ribonucleotides may be employed, which may be subjected to alkaline hydrolysis or glycol cleavage to provide for cleavage of the splinker. Any other linker may be employed for crosslinking the two complementary sequences, such as a polypeptide, which may be subjected to enzymatic hydrolysis, e.g., dilysine or diarginine, or other cleavable link, which may be introduced during the synthesis of the splinker by known means.

The splinker may include an internal single strand sequence of one or more mismatched bases, usually not more than about 10, usually not more than about 6 bases, so as to define a hairpin, or may be joined by non-nucleotide molecules, as indicated above.

The splinkers may be prepared by any convenient synthesis, such as using phosphites, phosphoramidites, or the like. The particular manner in which the splinkers are prepared is not critical to the subject invention and depending upon the nature of the linking group, which joins the two strands, different techniques may be employed.

The DNA fragments to be sequenced may be prepared in a variety of ways. The DNA fragments may be as a result of mechanical shearing such as sonication, endonuclease cleavage by one or more restriction enzymes, or other technique which provides for fragments of the desired size. Fragments which are employed will usually be at least about 0.1kb, more usually at least about 0.2kb, and preferably at least about 0.5kb, and may be 10kb or more, usually not greater than about 5kb. The fragments will usually be dephosphorylated with a phosphatase, e.g., alkaline phosphatase, so as to prevent any oligomerization of the fragments.

The DNA fragments to be sequenced may then be joined to splinkers. Ligation will follow conventional techniques employing, for example, T4 ligase. In blunt end ligations, the splinker will usually be employed in at least about a 20:1, preferably a 30:1 ratio of splinker ends to fragment ends, usually not more than about 100:1, while for sticky end ligations the splinker to fragment ratio will be at least about 10:1, and not more than about 50:1, more usually about 15-30:1.

The reaction time will vary with the concentrations, amount of enzyme, and the like, generally ranging from about 1-12hr. After sufficient time for completion, the reaction may be terminated by extraction with phenol, by itself or in combination with Sevag, followed by ethanol precipitation. For those DNA fragments having symmetrical termini where both ends terminate with splinkers, the fragments may be subjected to restriction, so as to provide two fragments terminating in splinkers. The fragments may then be separated, if necessary, and the terminus dephosphorylated.

Alternatively, the DNA sample may be digested with two different restriction enzymes to provide fragments having non-symmetrical ends, where two different splinkers may be employed, each complementary to a different end of the DNA fragment and each having a linking group which may be cleaved independent of cleavage of the other linking group. For example, each of the splinkers may have a complementary sequence which is a cleavage site for a different restriction enzyme. In this manner, each of the strands may be separately sequenced so as to provide for verification of the sequence.

The splinker-linked ds DNA fragment is then denatured to provide a ss fragment joined to the double-stranded splinker. A wide variety of denaturing agents are known and may be employed, e.g., 0.5M NaOH - 1.5M NaCl, heat at 100°C, or the like. With heat denaturation and sequencing may take place in the same medium. With chemical denaturation, the denaturation medium will usually be changed, either isolating the ss DNA or diluting the denaturation medium with a different medium which provides the desired sequencing medium.

The particular manner in which the sequencing is performed is not critical to this invention, so long as strands of different lengths are obtained, where the terminal nucleotide can be identified. Enzymes which may be employed for copying of the template strand include reverse transcriptase, e.g., AMV reverse transcriptase or DNA polymerase I, large fragment, which is commercially available as isolated from *E. coli*. The conditions for formation of the template are conventional and do not require description here, but may be found in the Experimental section. Mixtures of dideoxy-deoxy nucleotide mix are commercially available and may be obtained with radioactive labels, although as indicated previously, the splinker may be a source for the label. Alternatively, the Maxam-Gilbert technique may be employed. See, Maniatis *et al.*,

supra. Once the sequencing reaction products have been obtained, it may be resolved in accordance with conventional ways employing buffer gradient gels as particularly described in the Experimental section.

The splinker-linked DNA fragments may then be sized by a variety of ways, such as electrophoresis, gradient centrifugation, and the like. One technique which may be employed with advantage for sequences of greater than about 0.5kbp is referred to as the freeze-squeeze method (Tautz and Renz, Anal. Biochem. (1983) 132:14-19). The nucleic acids thus recovered from the size separation may be used directly for dideoxy sequencing.

The splinkers may also be used for the preparation of probes, either labeled or unlabeled. The probes may be partial fragments which would be size separated or a complete complementary strand where dNTPs are used in the absence of ddNTPs. The original strand may also be used as a probe, where both ends of a ds DNA are linked to splinkers prior to denaturation. The complementary region serving as the probe will usually have at least 10nt, usually at least 14nt, more usually at least about 30nt, and maybe 1000nt or more.

The following examples are offered by way of illustration and not by way of limitation.

## EXPERIMENTAL

Materials. T4 polynucleotide kinase and ligase were prepared as described previously (Panet et al., Biochemistry (1973) 12:5050-5055. Restriction endonucleases were purchased from New England Biolabs. Calf intestinal phosphatase (Grade 1 for immunoassay) and E. coli DNA polymerase I, large fragment were obtained from Boehringer Mannheim. AMV reverse transcriptase was purchased from Life Sciences, Inc. The E. coli DNA polymerase I, large fragment, primer extensions were carried out using a sequencing kit (catalogue number 1256) from PL Biochemicals. Dideoxy and deoxynucleoside triphosphates were also obtained from PL-Biochemicals. All $^{32}P$ labeled deoxynucleoside triphosphates were purchased from Amersham. ϕX174 was obtained from Gibco Canada (BRL).

Synthesis and purification of splinkers. Splinkers were synthesized on an Applied Biosystems Model 380A DNA synthesizer using phosphoramidite chemistry (Beaucage and Carruthers, Tetra. Lett. (1981) 22:1859-1862). Step yields were greater than 99% and the resulting oligodeoxynucleotides were purified by preparative polyacrylamide electrophoresis. Five $A_{260}$ units were applied to a 2cm well on a 20% polyacrylamide gel which contained 8M urea (1.2mm thick by 30cm long). The gel was run at 800 volts until the bromophenol blue was the bottom. The bands were visualized by short wave UV shadowing, excised, and subsequently electroeluted into Spectrapor dialysis tubing (Spectrum Medical Industries, MW cut-off 2000) in 0.5X TBE buffer [(5X TBE: 54g Trizma base, 27.5g boric acid plus 4.65g EDTA (disodium salt) per liter] at 75mA for 2hr. The gel slice was then removed and the current was reversed for 1min. The buffer was subsequently removed from the dialysis bag and its volume was reduced to 50μl with n-butanol. The nucleic acids were precipitated at -70°C following the addition of an equal volume of 5M ammonium acetate and 800μl 95% ethanol. The pellet was then resuspended in 100μl of 2.5M ammonium acetate (pH 5.5), insoluble matter was removed, and the nucleic acid was precipitated as before. Splinkers were 5' end labeled, using T4 polynucleotide kinase and [$\gamma$-$^{32}P$]ATP (Chaconas and van de Sande, Methods Enzymol. (1980) 65:75-85) immediately prior to ligation. Excess triphosphate was removed by dialysis and the splinkers were lyophilized.

<u>Preparation of restriction fragments for splinker sequencing</u>. Fragments for sequencing were obtained by digestion of plasmid with the appropriate restriction enzyme(s) according to the manufacturer's instructions. After the digestions were complete the reactions were brought to 50µl by the addition of 5µl of 10X CIP buffer (0.5M Tris-HCl, pH 9.0, 10mM $MgCl_2$, 1mM $ZnCl_2$, 10mM spermidine) and double distilled (dd) $H_2O$. Ends were dephosphorylated with the addition of 1µl of calf intestinal phosphatase (10mg/ml) as outlined by Maniatis et al., (1982) Molecular Cloning: A Laboratory Manual (Cold Spring Harbor Laboratory, Cold Spring Harbor, NY).

Blunt- or sticky-ended splinkers were ligated to the restriction fragments in 10µl reactions containing 50mM Tris-HCl, pH 7.4, 10mM $MgCl_2$, 10mM dithiothreitol (DTT), 10mM spermidine, and 1mM ATP at 20°C. In blunt-end ligations, splinker I was added to a concentration of 5µM, usually at a 50:1 ratio of splinker ends to fragment ends and incubated for 6hr with 3 units (Weiss et al., J. Biol. Chem. (1968) 243:4543-4549) of T4 DNA ligase. In sticky-end ligations, splinker II was added at a ratio of 20:1 in terms of 5' phosphate ends and reacted for 2hr with 1 unit of T4 DNA ligase. The reactions were terminated, by extraction with phenol/Sevag and the splinker ligated fragments recovered by ethanol precipitation. A second restriction cut was made and the resulting fragments containing a single splinker at one end were separated by electrophoresis. Alternatively, plasmids were digested with an additional restriction enzyme(s) to yield non-symmetrical ends, then dephosphorylated before the appropriate splinker was ligated to its matching end.

Fragments larger than 0.5kb were electrophoresed and resolved on agarose gels and purified by a modified freeze-squeeze method (Tautz and Renz, Anal. Biochem. (1983) 132:14-19). In brief, bands were localized by ethidium bromide fluorescence and a small channel was cut below the band which was plugged with 0.5% agarose. The plug was allowed to set for 10min at -20°C. The DNA was then electrophoresed into the 0.5% agarose plug, the plug was excised, and placed in a 500μl snap-cap tube which contained a siliconized glass wool plug at the bottom. Then, 400μl of 2X SET buffer (0.4M Tris-HCl, pH 7.8, 2mM EDTA, 0.3M NaCl) was added and the resulting mixture incubated at 20°C for 30min. The tube and contents were then frozen in liquid $N_2$ and subsequently the bottom of the tube was pierced with a 26 gauge needle. While the gel slurry was still frozen, the 500μl tube was placed in a 1.5ml tube and the nucleic acids were squeezed from the agarose by centrifugation in an Eppendorf microfuge for 5min. If any pieces of the gel were still visible the tube was refrozen in liquid $N_2$ and then centrifuged again. The flowthrough was extracted with n-butanol in order to reduce the volume to 200μl. The nucleic acids were then precipitated at -70°C following the addition of 0.33 volumes of 5M potassium acetate and 4 volumes of ethanol. The precipitate was subsequently dissolved in distilled water and dialyzed. The volume of the dialysate was reduced to 50μl by extraction with n-butanol. To this solution, an equal volume of 5M ammonium acetate, pH 5.5 was added. Insoluble material was removed and nucleic acid precipitated for 15min at -70°C after the addition of 800μl of 95% ethanol. The nucleic acids were pelleted by centrifugation, washed with 95% ethanol and then dried. Restriction fragments less than 0.5kb were separated on 8% polyacrylamide gels and localized either by autoradiography or ethidium bromide fluorescence. The bands of interest were cut out and the fragments electroeluted by the procedure described for the splinker (above). The nucleic acids thus recovered from the agarose or acrylamide gels were used directly for dideoxy sequencing.

Splinker sequencing. The splinker-primed template was suspended in 10μl of BamHI digestion buffer (50mM Tris-HCl, pH 7.5, 7mM $MgCl_2$, 60mM NaCl, 7mM DTT) heated at 100°C for 3min, subsequently placed in an ice bath for 5min, then left at 21°C for 15min. Copying of the template strand was initiated by the addition of AMV reverse transcriptase (RT) or *E. coli* DNA polymerase I, large fragment.

A sequencing kit containing premixed dideoxy-deoxy mixes and chase solution was used for dideoxy sequencing with *E. coli* DNA polymerase, large fragment. In brief, lyophilized [α-$^{32}$P]dATP (50μCi, 800Ci/mmole) was resuspended in the heat-chilled splinker-primed template. To four 500μl Eppendorf tubes labeled A, C, G and T, 3μl of the appropriate dideoxy mixes were added. The splinker-fragment/[α-$^{32}$P]dATP mixture (2μl) was then pipetted onto the side wall of each tube along with *E. coli* DNA polymerase I, large fragment (1μl, 1 unit/μl) and then the contents of the tubes were mixed by centrifugation. The reactions were incubated for 15min at 20°C. Chase solution (1μl) was then added and the reactions continued for an additional 15min. They were terminated by twice freezing the reaction tubes in liquid $N_2$ then heating them at 65°C for 3min. The splinker was then removed following the addition of 0.7μl of 10X EcoRI digestion buffer (0.5M Tris-HCl, 0.1M $MgCl_2$, 0.5M NaCl, 10mM DTT) containing 4 units/μl EcoRI by incubating the resulting mixture for 30min at 37°C. Splinker digestion was terminated by the addition of 5μl of stop solution (90% formamide in 1X TBE buffer with tracking dyes) or by freezing in liquid nitrogen.

BAD ORIGINAL

The protocol for chain termination sequencing with AMV reverse transcriptase was adapted from Karathanasis, supra. Lyophylized [α-$^{32}$P]ATP (50μCi, 800Ci/mmole) was resuspended in 3.5μl of 1X RTP buffer (10X RTP: 0.5M Tris-HCl, pH 8.5, 0.5M NaCl, 50mM $MgCl_2$ 20mM DTT) and 1.5μl of AMV reverse transcriptase (27 units) was added. This solution was combined with the heat-chilled splinker-template and 3μl of this mixture was added to 4 Eppendorf tubes labeled A, C, G and T. The 2μl of the appropriate dideoxy-deoxy nucleotide mix [5μM ddATP or 32.5μM ddGTP or 63.5μM ddTTP or 31.3μM ddCTP mixed 1:1 with RTP mix (20μl of each 0.5mM solution of dTTP, dCTP, dGTP, plus 20μl of 10X RTP buffer)] was added and the contents of the tubes were mixed by centrifugation. The reactions were then incubated at 42°C for 15min. Then 3μl of All Chase (12μl of 0.5mM solution of dATP, dCTP, dGTP, and dTTP plus 5.5μl of 10X RTP plus 27 units of AMV reverse transcriptase) was added. The reactions were subsequently completed by a second incubation at 42°C for 15min. They were then terminated by twice freezing the reaction tubes in liquid $N_2$ followed by heating them at 65°C for 3min. The splinker was then removed by the addition of 0.8μl of 10X EcoRI digestion buffer containing 4 units/μl EcoRI and incubating the resulting mixture for 30min at 37°C. Splinker digestion was terminated by the addition of 6μl of stop solution or by freezing in liquid nitrogen.

Sequencing reaction products were resolved on 85cm x 20cm x 0.4mm 8% polyacrylamide (19:1 acrylamide/bisacrylamide) buffer gradient gels (Biggin et al., Proc. Natl. Acad. Sci. USA (1983) 80:3963-3965) containing 8M urea. The gel and running buffers were made with pH 9.0 TBE buffer (10X TBE; 162gm Trizma base, 27.5gm boric acid, and 9.3gm EDTA per liter). The long gradient gels were poured in two steps. Initially 10mls of lower gel solution (2.5X TBE buffered acrylamide plus urea) were poured. After letting the lower gel settle to form an even surface, the upper gel solution (0.5X TBE buffered acrylamide plus urea) was layered overtop by pouring it along the side spacer. The gel was allowed to polymerize for approximately 1hr then pre-electrophoresed at 1.5 kilovolts for 2hr. Samples containing stop buffer were heated at 100°C for 3min, then immediately applied to the gel. Electrophoresis was for 15hr at 2 kilovolts.

In accordance with the prior procedures, two splinkers were prepared having the following sequences:

```
I      T  T G A G A A T T C T     3'
       T  T C T C T T C C G A     5'

II     T  T T A G A A T T C T         3'
       T  T A T C T T A A G A G C     5'
```

Each of the linkers has an EcoRI restriction site, a loop of four thymidines, with linker I having a blunt end and linker II having an overhang of two bases, GC. The loops serve to stabilize the binding of the complementary strands, so as to define a hairpin. The hairpin structures of the splinkers are quite stable (i.e., Tm of 57°C in 10mM NaCl for splinker II, with digestion of splinkers I and II by EcoRI providing the expected dinucleotide and tetranucleotide fragments. Splinker II ($^{32}$P-labeled) was joined to 352 and 4009 dephosphorylated base pair fragments from ClaI/BamHI digestion of pBR322. pBR322 (2μg) was digested with 2 units of ClaI and 2 units of BamHI to completion. The 5' ends were dephosphorylated using alkaline phosphatase. Splinker II was then added using T4 DNA ligase.

The joining of splinker to the ClaI digested end of the 352 base pair fragment was observed by a shift in mobility and the presence of radioactivity in the shifted band. The presence of $^{32}P$ in the large fragment also indicated that the splinker had added to the ClaI and of the large fragment. The splinker-352 fragment was recovered from the gel by electroelution and the splinker-4009 was recovered from a 1% agarose by freeze-squeeze. These splinker/DNA fragments were used in primer extension reactions as described previously in the presence of dideoxy chain terminators. The chain terminated extension products were released from the template strand by EcoRI digestion and were resolved on an 8% polyacrylamide buffer gradient sequencing gel. AMV reverse transcriptase was employed to copy the small 352bp fragment and the nucleotides determined for nucleotides 48-190 were shown to be identical to that sequence of pBR322. Both AMV reverse transcriptase and E. coli DNA polymerase I were employed for copying the 4009bp template. Some background sequence was observed in the lower portion (approximately the first 20 nucleotides) of the sequencing gel. This was due to a residual activity of the AMV reverse transcriptase or E. coli DNA polymerase I, large fragment, which was effecting repair during the EcoRI release of the primer.

The blunt-ended splinker I was joined to a dephosphorylated fragment (1130bp) generated by HpaI/XhoI digestion of $\phi$X174 replicative form. The isolated splinker-fragment was subsequently used for primer extension with either of the two enzymes described above. In this case the enzymes were inactivated prior to the EcoRI release step of the primer extended products. The $\phi$X174 sequence from nucleotide 275 was read with ease.

The primer-linker approach of this invention to DNA sequencing provides a convenient method for chain termination sequencing of DNA fragments. It allows for completion of a shotgun sequencing project from large sized inserts by splinker sequencing from

BAD ORIGINAL

the most distal (5') and appropriate newly determined restriction sites. It alleviates the necessity to subclone these large sized inserts as a series of small DNA fragments, some of which may be under or not represented. Since the primer strand is always covalently attached to the template strand, this method will also lend itself to an alternative "walking" approach to kilobase sequencing. Release of DNA extension areas distal from the splinker site will permit the sequence acquisition while the proximal extension reactions remain part of a covalently linked complementary DNA duplex. Furthermore, the use of unsymmetrical splinkers containing different restriction enzyme sites permits the simultaneous sequencing of both strands and hence the complete sequence for any duplex DNA.

Although the foregoing invention has been described in some detail by way of illustration and example for purposes of clarity of understanding, it will be obvious that certain changes and modifications may be practiced within the scope of the appended claims.

WHAT IS CLAIMED IS:

1. In a method for chain termination sequencing of a DNA sequence, which comprises replicating a template chain with mixtures of nucleotides, where each of the mixtures has a different composition resulting in a mixture of partial sequence strands terminating at different nucleotides in the sequence, separating the partial sequence strands into individual length strands and determining the nucleotide sequence as a result of the site of termination of the strands, the improvement which comprises:

joining only one of the termini at at least one of the ends of a double-stranded DNA fragment to be sequenced to a splinker, wherein said splinker is characterized by having a complementary region of at least four base pairs and a selectively cleavable link;

denaturing the splinker-linked double-stranded DNA to provide for a splinker-linked single-stranded DNA as a template fragment, where said splinker acts as a primer; and

enzymatically extending the primer with said mixtures of nucleotides for sequencing.

2. A method according to Claim 1, wherein said selectively cleavable link is a restriction site.

3. A method according to Claim 1, wherein said selectively cleavable link is a ribonucleotide.

BAD ORIGINAL

4. In a method for chain termination sequencing of a DNA sequence which comprises replicating a template chain with mixtures of nucleotides where each of the mixtures has a different dideoxy nucleotide, resulting in a mixture of partial sequence strands terminating at different nucleotides in the sequence, separating the partial sequence strands by electrophoresis into individual strand lengths and determining the nucleotide sequence as a result of the site of termination of the strands, the improvement which comprises:

joining a double-stranded DNA fragment to be sequenced having hydroxyl termini to a splinker, wherein said splinker is characterized by having two complementary regions proximal to opposite termini capable of duplexing to form a double strand of at least five base pairs, each of said complementary regions joined by a single strand of at least three nucleotides, so that the entire splinker defines a hairpin, said duplex region defining a restriction site;

denaturing the splinker-linked double-stranded DNA to provide for a splinker-linked single-stranded DNA as a template fragment, where said splinker acts as a primer; and

enzymatically extending the primer with said chain terminating mixtures of nucleotides for sequencing.

5. A method according to Claim 4, wherein said splinker has a label which remains bound to said partial sequences after restriction at said splinker restriction site.

6. A composition of matter comprising a single-stranded DNA of at least 12 nucleotides comprising complementary regions capable of duplexing proximal to the termini joined by a single-stranded sequence of at least three nucleotides.

7. A composition of matter according to Claim 6, wherein said single strand is of up to and including 30 nucleotides and wherein said complementary region is at least five base pairs and at least one of the complementary regions is at the terminus.

8. A composition of matter according to Claim 7, wherein the 5' terminus of said single-stranded sequence is phosphorylated.

9. A composition of matter according to Claim 6, wherein the 5' or 3' strand has a two nucleotide overhang, into at least one 3' overhang of a restriction site.

10. A probe for detecting a nucleic acid sequence of interest comprising a composition of matter according to Claim 6 joined at one terminus to a DNA sequence complementary to said sequence of interest.